# EUROPEAN PATENT APPLICATION

(11) **EP 1 277 452 A1**
(43) Date of publication of application: **22.01.2003**
(21) Application number: 02013549.7
(22) Date of filing: 19.06.2002
(51) Int. Cl.: A61F 5/455

(54) **Device for female urination in erect position**

(30) Priority: 27.06.2001 IT BO20010404
(71) Applicant: Ganzerli, Luigi, 41036 Medolla (Modena) (IT)
(72) Inventor: Ganzerli, Luigi, 41036 Medolla (Modena) (IT)
(74) Representative: Modiano, Guido, Dr.-Ing.

(57) **Abstract**

A device for female urination in erect position made of layered material which is substantially frustum-shaped, comprising a film of thermoplastic material that is water-soluble at ambient temperature and in a short time suitable to allow the dissolving of said device after use.

## Description

The present invention relates to a device for female urination in erect position.

It is known that various devices for assisting female urination are commercially available and are indispensable especially when the hygiene conditions of toilets are insufficient. In this case, a woman, in order to avoid direct contacts with the toilet, has to resort to makeshift methods, such as for example placing sheets of toilet paper on the bowl rim and other methods that can prevent women from making contact with the bowl rim.

The background art has proposed various devices to allow urination of women in an erect position.

US-2,878,486 discloses a device for female urination that is obtained from a sheet of paper folded into a funnel-like shape and provided with an end that is anatomically adapted to the external region of female genitalia and another end that allows the outflow of urine.

In order to use such device, a woman, in erect position, simply has to open her legs slightly and place the device on her genitalia. After this preparatory step, the woman is ready to urinate.

Moreover, US-5,408,703 in the name of Cicio discloses a frustum-shaped device for female urination that receives the urine at a first end and voids it with the other end by means of another opening.

Although these devices solve the hygiene problem related to physical contact between the woman and the toilet, they do not solve another equally important problem, i.e., the disposal of the device. Although most of these devices are biodegradable, they are abandoned in ordinary toilet trash after use. This inevitably causes not only unpleasant odors but also hygiene problems caused by the proliferation of bacterial forms that are dangerous to human health.

The aim of the present invention is to solve the above described problems by using a device for female urination that in addition to avoiding physical contact of the woman with the toilet does not cause hygiene problems after use.

Within this aim, an object of the present invention is to provide a device that is simple, easy to produce in practice, safe in use and effective in operation.

Another object of the present invention is to provide a device that can be manufactured easily in various sizes.

This aim and these and other objects that will become better apparent hereinafter are achieved by a device for female urination in erect position made of layered material which is substantially frustum-shaped, characterized in that it is constituted by a film of thermoplastic material that is water-soluble at ambient temperature and in a short time suitable to allow the dissolving of said device after use.

Further characteristics and advantages of the present invention will become better apparent from the following detailed description of a preferred but not exclusive embodiment of a device for female urination in erect position, illustrated only by way of non-limitative example in the accompanying drawings, wherein:
Figure 1 is a perspective view of the device according to the present invention;
Figure 2 is a view of the device spread out flat;
Figure 3 is a view of an operating position of said device.

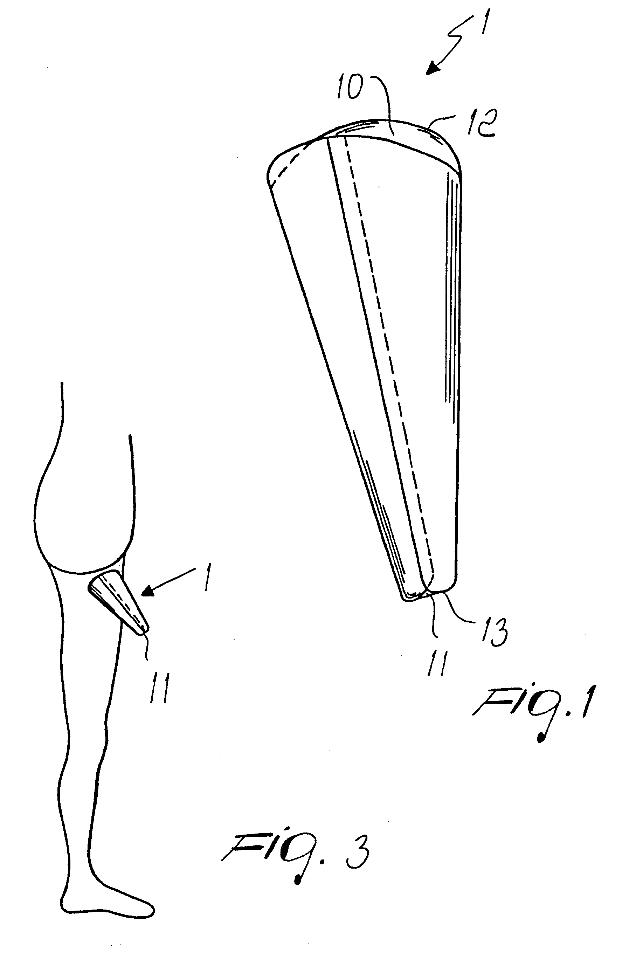

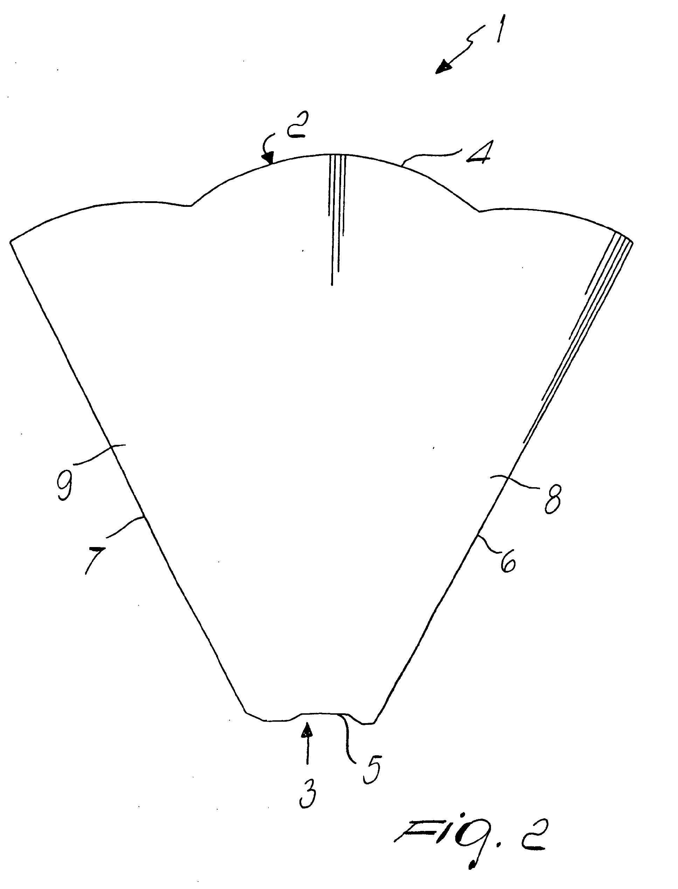

With reference to the figures, the reference numeral 1 generally designates the device according to the invention.

The device 1 is constituted by a film made of thermoplastic material based on polyvinyl alcohol that makes it water-soluble. The polyvinyl alcohol base is such as to allow the device to dissolve in water in a short time at temperatures substantially equal to ambient values. In practice, the dissolving time is optimized around the time of a few refills of the toilet cistern.

The film of the device 1 has, when spread out flat, substantially the shape of an isosceles trapezoid obtained by automated die-cutting from a roll of film. The trapezoid has, on its longer parallel side 2 and on its shorter parallel side 3, shaped portions 4 and 5. On the oblique sides 6 and 7, it forms respective flaps 8 and 9, which are glued by means of water so as to form the frustum-like shape of the device 1.

The device 1 forms a larger opening 10 and a smaller opening 11, the respective edges 12 and 13 of which have a shape that depends on the shaped portions 4 and 5. In particular, the edge 12 has an anatomically contoured shape that is complementary to the outer part of female genitalia.

In practical operation, a woman, in order to urinate in an erect position, has to open her legs slightly and place the device 1 with the part provided with the larger opening 10 at the female genitalia.

During urination, the urine is guided by the device and released into the toilet bowl through the smaller opening. Once urination has been performed, the device is thrown into the water of the toilet bowl. After a certain time, substantially equal to the time of a few refills of the cistern, the device dissolves.

In order to reduce the thickness of the film used and therefore speed up the crumpling time during the final step of immersion in water after use, a thin sheet of cellulose-based paper is coupled to the film of water-soluble thermoplastic material. Advantageously, the thin paper sheet is arranged externally and is intimately bonded to the thermoplastic material by impregnation with water and optionally adhesives of the kind based on fish glue, vegetable flours, or the like.

The thin paper sheet, in addition to producing a cost reduction, is also capable of improving the characteristics of strength and elasticity of the structure, making it easier and more straightforward to compress and manually draw toward each other the two mutually opposite folds that the device has in the condition for carrying and packaging, in order to open it into the configuration for use.

Advantageously, the material that constitutes the film is a calendered water-soluble vinyl polymer, while the thin sheet of paper can be cellulose-based, similar to toilet paper with a thickness between 50 and 200 microns.

It has thus been observed that the invention achieves the intended aim and objects.

The invention thus conceived is susceptible of numerous modifications and variations, all of which are within the scope of the inventive concept.

In particular, it is stressed that the coloring of the thermoplastic film may be any (neutral or colored).

All the details may further be replaced with other technically equivalent ones.

In practice, the materials used, as well as the shapes and the dimensions, may be any according to requirements without thereby abandoning the scope of the protection of the appended claims.

The disclosures in Italian Patent Application No. BO2001A000404 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A device (1) for female urination in erect position made of layered material which is substantially frustum-shaped, **characterized in that** it is constituted by a film of thermoplastic material that is water-soluble at ambient temperature and in a short time suitable to allow the dissolving of said device after use.

2. The device according to claim 1, **characterized in that** said thermoplastic film is based on polyvinyl alcohol.

3. The device according to claim 1 and 2, **characterized in that** said device is meant to be released into the water of the toilet.

4. The device according to the preceding claims, **characterized in that** the dissolving time is substantially within the time of a few refills of the toilet cistern.

5. The device according to the preceding claims, **characterized in that** said film is substantially trapezoidal.

6. The device according to the preceding claims, **characterized in that** the frustum-like shape is obtained by gluing, by means of water, opposite oblique sides (6,7) of said trapezoidal film.

7. The device according to the preceding claims, **characterized in that** said trapezoid is isosceles.

8. The device according to the preceding claims, **characterized in that** said trapezoid is obtained by die-cutting.

9. The device according to the preceding claims, **characterized in that** said trapezoid has respective shaped portions (4,5) on its larger and smaller parallel sides (2,3).

10. The device according to the preceding claims, **characterized in that** it comprises a larger opening (10) and a smaller opening (11), said larger opening being suitable to be rested against the female genitalia and said smaller opening being suitable to discharge the fluid during urination into the toilet bowl.

11. The device according to one or more of the preceding claims, **characterized in that** a thin sheet of cellulose-based paper is coupled to the film of water-soluble thermoplastic material.

12. The device according to one or more of the preceding claims, **characterized in that** said thin sheet of paper is arranged on the outside of the film of thermoplastic material and is intimately bonded to the thermoplastic material by impregnation with water and with adhesives.

13. The device according to claim 12, **characterized in that** said adhesives are of the type based on fish glue, vegetable flours or the like.

14. The device according to one or more of the preceding claims, **characterized in that** the material that constitutes the film is a calendered water-soluble vinyl polymer and the thin sheet of paper is cellulose-based, of the same kind as toilet paper with a thickness between 50 and 200 microns.
